# EUROPEAN PATENT APPLICATION

(11) **EP 0 755 931 A1**
(43) Date of publication of application: **29.01.1997**
(21) Application number: 96109238.4
(22) Date of filing: 10.06.1996
(51) Int. Cl.: C07D 401/10, A61K 31/44

(54) **Dihydropyridine and pyridine derivatives and process for their preparation**

(30) Priority: 27.07.1995 GB 9515445
(71) Applicant: PHARMACIA & UPJOHN S.p.A., 20152 Milano (IT)
(72) Inventor: Cozzi, Paolo, 20133 Milan (IT); Di Salle, Enrico, 20124 Milan (IT); Rossi, Arsenia, 24044 Dalmine, (Bergamo) (IT)
(74) Representative: Ferrario, Vittorino

(57) **Abstract**

A steroidal aromatase inhibitor compound of the following formulae (I) and (II) 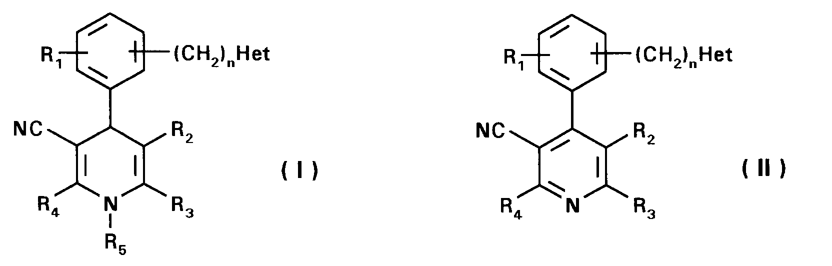 wherein:
Het is 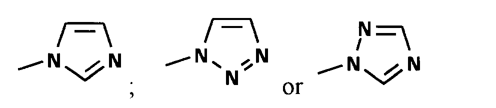 n is zero or 1;
R₁ is hydrogen, halogen, trihalomethyl or C₁-C₃ alkyl;
R₂ is cyano or COOR, wherein R is C₁-C₆ alkyl either unsubstituted or omega substituted by C₁-C₃ alkoxy;
each of R₃ and R₄, which may be the same or different, is a C₁-C₄ alkyl group, and
R₅ is hydrogen or a C₁-C₄ alkyl group, and the pharmaceutically acceptable salts thereof;
is provided.

## Description

The present invention relates to new imidazolyl and triazolyl derivatives of 4-phenyl substituted 1,4-dihydropyridines and pyridines, to a process for their preparation and to pharmaceutical compositions containing them.
WO 90/06923 and EP-A-533504 disclose certain imidazolyl derivates of 4-phenyl substituted 1,4-dihydropyridine. The compounds according to the prior-art international application are thromboxane A₂ synthase inhibitors and calcium antagonists, whereas those according to the European application are useful for re-sensitizing multiple drug resistant cells to chemotherapeutic agents.

The imidazolyl and triazolyl derivatives subject of the present invention are compounds of the following formulae (I) and (II). 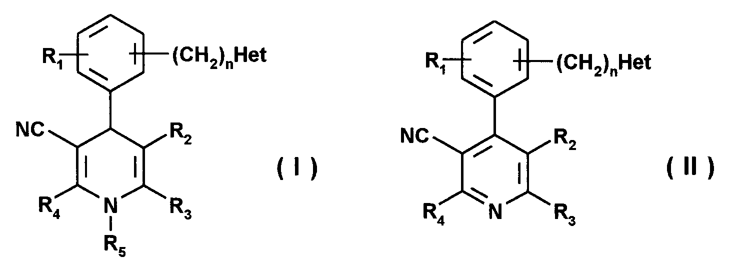 wherein:
Het is 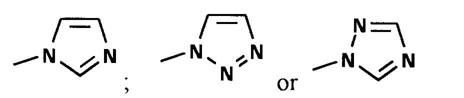 n is zero or 1;
R₁ is hydrogen, halogen, trihalomethyl or C₁-C₃ alkyl;
R₂ is cyano or COOR, wherein R is C₁-C₆ alkyl either unsubstituted or omega substituted by C₁-C₃ alkoxy;
each of R₃ and R₄, which may be the same or different, is a C₁-C₄ alkyl group, and R₅ is hydrogen or a C₁-C₄ alkyl group.
The invention includes also the pharmaceutically acceptable salts of the compounds of formula (I) and (II) as well as all the possible isomers and stereoisomers thereof and their mixtures. Also the pharmaceutically acceptable bio-precursors (otherwise known as pro-drugs) of the compounds of formula (I) and (II), i.e. compounds which have a different formula to formula (I) and (II) above but which nevertheless upon administration to a human being are converted directly or indirectly in vivo into a compound of formula (I) and (II), are included within the scope of the invention.
Phamaceutically accetable salts of the compounds of formula (I) are, especially, acid addition salts with inorganic, e.g. nitric, hydrochloric, hydrobromic, sulphuric, perchloric and phosphoric acids, or organic, e.g. acetic, propionic, glycolic, lactic, oxalic, malonic, malic, maleic, tartaric, citric, benzoic, cinnamic, mandelic, fumaric, methanesulfonic and salicylic acids.
The reciprocal substitution position on the phenyl ring of the (CN₂)ₙHet group and 4-dihydropyridyl or 4-pyridyl moiety is preferably meta or para.
The alkyl groups may be branched or straight chain groups.
A C₁-C₃ alkyl group is preferably methyl, ethyl or n-propyl.
A C₁-C₆ alkyl group is preferably a C₁-C₄ alkyl group, in particular methyl, ethyl, n-propyl, isopropyl or isobutyl.
A C₁-C₃ alkoxy group is, preferably, methoxy or ethoxy, particularly methoxy.
A halogen is, preferably, chlorine, bromine or fluorine, in particular chlorine or fluorine.
A trihalomethyl is preferably a trifluoromethyl.

A preferred class of compounds according to the invention are the compounds of formula (I) and (II) wherein
n is zero, R₁ is hydrogen, Het is 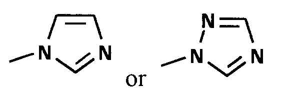 and R₂, R₃, R₄, and R₅ are as defined above; and their pharmaceutically acceptable salts.

Specific examples of preferred compounds, according to the invention, are:
4-(3-1H-imidazol-1-yl-phenyl)-2,6-dimethyl-1,4-dihydropyridine-3,5-dicarbonitrile;
4-(4-1H-imidazol-1-yl-phenyl)-2,6-dimethyl-1,4-dihydropyridine-3,5-dicarbonitrile;
4-(3-1H-imidazol-1-yl-phenyl)-2,6-dimethyl-1,4-dihydropyridine-3-carbonitrile-5-carboxylic acid ethyl ester;
4-(3-1H--imidazol-1-yl-phenyl)-2,6-dimethyl-1,4-dihydropyridine-3-carbonitrile-5-carboxylic acid isopropyl ester;
4-[3-(1,2,4)triazol-1-yl-phenyl]-2,6-dimethyl-1,4-dihydropyridine-3,5-dicarbonitrile;
4-[3 -(1,2,4)triazol-1-yl-phenyl]-2,6-dimethyl-1,4-dihydropyridine-3-carbonitrile-5-carboxylic acid ethyl ester;
4-(3-1H-imidazol-1-yl-phenyl)-2,6-dimethylpyridine-3,5-dicarbonitrile;
4-(3-1H-imidazol-1-yl-phenyl)-2,6-dimethylpyridine-3-carbonitrile-5-carboxylic acid ethyl ester;
4-[3-(1,2,4)triazol-1-yl-phenyl]-2,6-dimethylpyridine-3,5-dicarbonitrile;
4-[3-(1,2,4)triazol-1-yl-phenyl]-2,6-dimethylpyridine-3-carbohitrile-5-carboxylic acid ethyl ester;
4-(3-1H-imidazol-1-yl-phenyl)-1,2,6-trimethyl-1,4-dihydropyridine-3,5-dicarboritrile; and
4-[3-(1,2,4)triazol-1-yl-phenyl]-1,2,6-trimethyl-1,4-dihydropyridine-3,5-dicarbonitrile;
either as single isomers or as a mixture thereof and the pharmaceutically acceptable salts thereof.
The compounds of formula (I) and (II) as defined above, and the salts thereof are hereafter defined as the compounds of the invention.

The compounds of the invention can be obtained by a process comprising:
a) reacting a compound of formula (III) 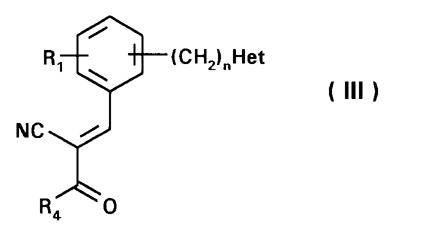 wherein
   R₁, Het, n, and R₄ are as defined above, with a compound of formula (IV) 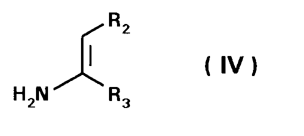 wherein
   R₂ and R₃ are as defined above, thus obtaining a compound of formula (I) in which R₅ is hydrogen; or
b) reacting a compound of formula (III), as defined above, with a compound of formula (V) 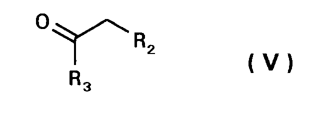 wherein
   R₂ and R₃ are as defined above, in the presence of an ammonium salt or hydroxide, thus obtaining a compound of formula (I) in which R₅ is hydrogen; or
c) reacting a compound of formula (VI) 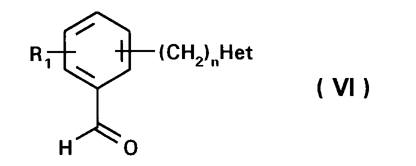 wherein R₁, n and Het are as defined above, with a compound of formula (IV), as defined above, and a compound of formula (VII), together, 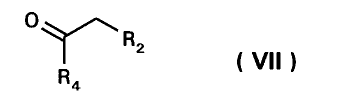 wherein R₂ and R₄ are as defined above and in at least one of the compounds (IV) and (VII) R₂ is cyano, thus obtaining a compound of formula (I) wherein R₅ is hydrogen; or
d) reacting a compound of formula (VI), as defined above, with a compound of formula (V), as defined above, and a compound of formula (VII), as defined above, together, wherein in at least one of the compounds (V) and (VII) R₂ is cyano, in the presence of ammonium salt or hydroxide, thus obtaining a compound of formula (I) wherein R₅ is hydrogen; or
e) reacting a compound of formula (VI), as defined above, with at least two equivalents of a compound of formula (IV) , as defined above, wherein R₂ is a cyano group, thus obtaining a compound of formula (I), wherein R₂ is cyano, R₃ and R₄, being as defined above, are the same and R₅ is hydrogen; or
f) alkylating a compound of formula (I), as defined above, wherein R₅ is hydrogen, thus obtaining a compound of formula (I) wherein R₅ is C₁-C₄ alkyl; or
g) oxidizing a compound of formula (I), as defined above, wherein R₅ is hydrogen, thus obtaining a compound of formula (II), as defined above; and, if desidered, converting a compound of the invention into another compound of the invention; and/or, if desired, converting a compound of formula (I) or (II) into a pharmaceutically acceptable salt thereof and/or, if desired, converting a salt into a free compound, and/or, if desired, separating a mixture of isomers of formula (I) or (II) into the single isomers.

The reactions described above under a), b), c), d) and e) can be performed by using known methods of the organic chemistry and, particularly, those typical of the chemistry of 1,4-dihydropyridines, such as those described e.g. by U. Eisner and Kuthan in Chem. Rev. 72, 1(1972) and by D.M. Stout and A.I. Meyers in Chem. Rev. 82, 223, (1982).
In particular, reactions such as those described under a), b), c) and d) may be carried out following the same basic procedure, e.g. by heating the reactants at a temperature ranging from about 50°C to about 150°C in a suitable inert organic solvent such as methanol, ethanol, isopropanol, dioxane, tetrahydrofuran, dimethylformamide, acetonitrile, dimethylsulfoxide, pyridine or their mixtures, or acetic acid.
The ammonium hydroxide used in processes b) and d) may be, for example, in the form of concentrated aqueous ammonia, while an ammonium salt may be, for instance, ammonium acetate.
Alkylation of a compond of formula (I) wherein R₅ is hydrogen, according to process variant f), may be carried out by reaction with a suitable C₁-C₄ alkyl halide, preferably the iodide, in the presence of a strong base, preferably sodium hydride at room temperature, under an inert gas atmosphere, e.g. nitrogen atmosphere, in an inert solvent e.g. dimethylformamide.

The reaction described under g) can be carried out using known methods in the organic chemistry and in particular known oxidative procedures converting 1,4 dihydropyridine ring into pyridine ring as those described e.g. in J. Med. Chem. 36, 2964, (1993).
Also the conversion of a compound of the invention into another compound of the invention can be carried out according to known methods. For instance alkylation process f) can be regarded as a conversion of a compound of the invention into another compound of the invention.
The optional salification of a compound of the invention as well as the conversion of a salt into the free compound and the separation of a mixture of isomers into the single isomers may be carried out by conventional methods.
A compounds of formula (III) may be prepared by reacting a compound of formula (VI), as defined above, with a compound of formula (VII), as defined above, wherein R₂ is a cyano group, following the well known procedure for the Knoevenagel reaction, such as, e.g., described by G. Jones in Org. Reactions, 15 (1967) pp. 204-599.
The process is preferably carried out by reacting compounds (VI) and (VII) in the presence of a suitable base, e.g. diethylamine or pyridine, in a suitable solvent, e.g. ethanol or benzene, at temperatures ranging approximately from room temperature to the reflux. The compounds of formulae (IV), (V) and (VII) are known compounds or may be prepared following usual procedures from known compounds.

The compounds of formula (VI) are known compounds too or may be prepared by known methods from known compounds e.g. by reducing the corresponding alkyl esters of formula (VIII) 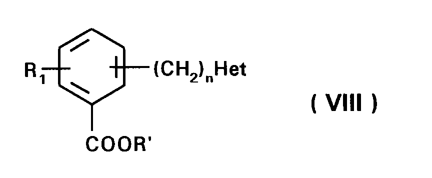 wherein
R₁, n and Het are as defined above and R' is a lower alkyl group, typically a C₁-C₄ alkyl group.
The reduction may be performed in the presence of a suitable reducing agent as, e.g., diisobutylaluminium hydride in a suitable solvent such as, e.g., diethylether or tetrahydrofuran, at temperatures ranging from about -80°C to the room temperature.
Alternatively, compounds of formula (VI) may be prepared by oxidation of the corresponding alcohol of formula (IX) 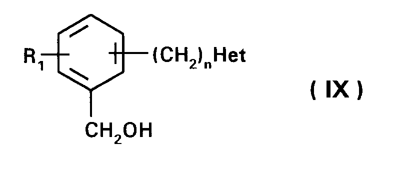 wherein
R₁, n and Het are as defined above.
The process of oxidation may be performed following well known procedures for converting a primary alcohol to the corresponding aldehyde, e.g. those described by J. March in Advanced organic Chemistry 1985, J. Wiley Publ.,pp.1057-1060. Moreover compounds of formula (VI) wherein n is zero may be prepared by oxidation of compounds of formula (X). 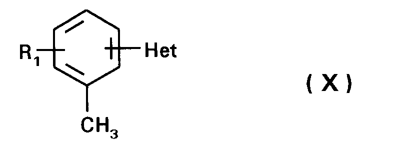 wherein R₁ and Het are as defined above.
The process of oxidation may be performed following known procedure, e.g. by use of chromic anhydride in acetic anhydride. Compounds of formulae (VIII), (IX) and (X) are known compounds or may be prepared following known procedures, e.g. those reported in J. Med. Chem. 36, 2964, (1993).

### PHARMACOLOGY

The compounds of the present invention are therapeutic substances, being inhibitors of the biotransformation of androgens into estrogens, i.e they are steroidal aromatase inhibitors.
Basic and clinical data indicate that aromatized metabolites of androgens, i.e. the estrogens, are the hormones involved in the pathogenic cellular changes associated with the growth of some hormone-dependent cancers, such as breast, endometrial and ovarian carcinomas.
Estrogens are also involved in the pathogenesis of benign prostatic hyperplasia.
Endogenous estrogens are ultimately formed from either androstenedione or testosterone as immediate precursors. The reaction of central importance is the aromatization of the steroidic ring A, which is performed by the enzyme aromatase. As aromatization is a unique reaction and the last in the series of steps in the biosynthesis of estrogens, it has been envisaged that an effective inhibition of the aromatase, resulting from compounds able to interact with the aromatizing steps, may have useful application for controlling the amount of circulating estrogens, estrogen dependent processes in reproduction, and estrogen-dipendent tumours.
The aromatase inhibitory activity of the compounds of the invention was demonstrated e.g. by employing the in vitro test described by Thompson and Siiteri (E.A. Thompson and P.K. Siiteri, J. Biol. Chem. 249, 5364, 1974) which utilizes the human placental microsomal fraction as enzyme source. In this test the aromatization rate of androstenedione into estrone was evaluated by incubating (1β-³H) androstenedione (50 nM) in the presence of NADPH with the enzyme preparation and by measuring the amount of³H₂O formed during 15 min incubation at 37°C.
The compounds, incubated at various concentrations, showed a relevant aromatase inhibitory activity.
For example the representative compounds of the invention 4-(3-1H-imidazol-1-yl-phenyl)-2,6-dimethyl-1,4-dihydropyridine-3,5-dicarbonitrile (internal code FCE 28718); ± 4-(3-1H-imidazol-1-yl-phenyl)-2,6-dimethyl-1,4-dihydropyridine-3-carbonitrile-5-carboxylic acid ethyl ester (internal code FCE 29013); 4-[3-(1,2,4)triazol-1-yl-phenyl]-2,6-dimethyl-1,4-dihydropyridine-3,5-dicarbonitrile (internal code FCE 29138) and 4-(3-1H-imidazol-1-yl-phenyl)-2,6-dimethylpyridine-3,5-dicarbonitrile (internal code FCE 28717) were found to exhibit a marked inhibitory activity of human placental aromatase, significantly more potent than that of the reference compound aminoglutethimide. These results are summarized in Table I.

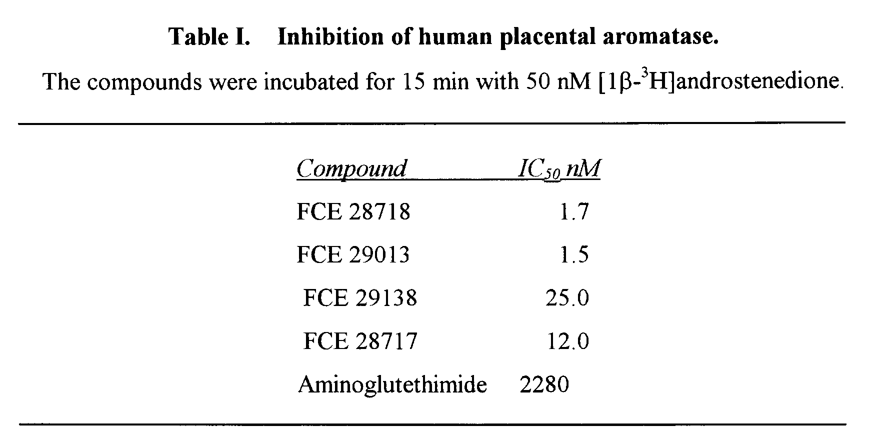

Thus the exemplified compounds are about between two and three order of magnitude more potent than reference compound aminoglutethimide, a drug used in the treatment of estrogen-dependent breast cancer (see e.g. C.R.C. Crit. Rev. Oncol. Hematol. 5, 361 (1986).
By virtue of their ability to inhibit aromatase and, consequently, to reduce estrogen levels, the compounds of the invention are therefore useful in mammals, including humans, in the treatment or prevention of various estrogen-dependent diseases, i.e. breast, endometrial, ovarian and pancreatic cancers, gynecomastia, benign breast disease, endometriosis, polycystic ovarian disease and precocious puberty. Another application of the compounds of the invention is in the therapeutic and/or prophylactic treatment of prostatic hyperplasia, a disease of the estrogen-dependent stromal tissue. The compounds of the invention can find also use for the treatment of male infertility associated with oligospermia and for female fertility control, by virtue of their ability to inhibit ovulation and egg nidation.
The compounds of the invention can be administered in a variety of dosage forms, e.g. orally, in the form of tablets, capsules, sugar or film coated tablets, liquid solutions or suspensions; rectally, in the form of suppositories; parenterally, e.g. intramusculary, or by intravenous injection or infusion. The dosage depends on the age, weight, conditions of the patient and administration route; for example, the dosage adopted for oral administration to adult humans for compound FCE 28718 may range from about 10 to about 150-200 mg pro dose, from 1 to 5 times daily.
The invention includes pharmaceutical compositions comprising a compound of the invention in association with a pharmaceutically acceptable excipient (which can be a carrier or diluent).
The pharmaceutical compositions containing the compounds of the invention are usually prepared following conventional methods and are administered in a pharmaceutically suitable form. For example the solid oral forms may contain, together with the active compound, diluents, e.g. lactose, dextrose, saccharose, cellulose, corn starch or potato starch; lubricants, e g. silica, talc, stearic acid, magnesium or calcium stearate, and/or polyethylene glycols; binding agents, e.g. starches, arabic gums, gelatin, methylcellulose, carboxymethylcellulose or polyvinyl pyrrolidone; disaggregating agents, e.g. a starch, alginic acid, alginates or sodium starch glycolate; effervescing mixtures; dyestuffs, sweeteners; wetting agents, such as lecithin, polysorbates, laurylsulphates; and, in general, non-toxic and pharmacologically inactive substances used in pharmaceutical formulations. Said pharmaceutical preparations may be manufactured in known manner, for example, by means of mixing, granulating, tabletting, sugar-coating, or film-coating processes. The liquid dispersions for oral administration may be e.g. syrups, emulsions and suspensions.
The syrups may contain as carrier, for example, saccharose or saccharose with glycerine and/or mannitol and/or sorbitol. The suspensions and the emulsions may contain as carrier, for example, a natural gum, agar, sodium alginate, pectin, methylcellulose, carboxymethylcellulose, or polyvinyl alcohol. The suspensions or solutions for intramuscular injections may contain, together with the active compound, a pharmaceutically acceptable carrier, e.g. sterile water, olive oil, ethyl oleate, glycols, e.g. propylene glycol, and if desired, a suitable amount of lidocaine hydrochloride. The solutions for intravenous injections or infusions may contain as carrier, for example, sterile water or preferably they may be in the form of sterile, aqueous, isotonic saline solutions. The suppositories may contain together with the active compound a pharmaceutically acceptable carrier, e.g. cocoa-butter, polyethylene glycol, a polyoxyethylene sorbitan fatty acid ester surfactant or lecithin.

The following examples illustrate but do not limit the present invention.

### EXAMPLE I

A mixture of 3-(1H-imidazol-1-yl)benzaldehyde (1.77g., 0.01 moles) and 3-aminocrotonitrile (1.86g., 0.023 moles) in glacial acetic acid (12ml) is refluxed for 5 hours. The reaction mixture is evaporated under vacuum and the crude product is purified by silica gel column (eluant: CH₂Cl₂ / EtOH = 95 / 5), yielding 1.01g. (34%) of
4-(3-1H-imidazol-1-yl-phenyl)-2,6-dimethyl-1,4-dihydropyridine-3,5-dicarbonitrile,
m.p. = 305-325°C.
Microanalysis:
Found: C 70.42; H 5.20; N 22.36
Calculated for C₁₈H₁₅N₅: C 71.70; H 5.02; N 23.24
¹H-NMR (200MHz, DMSO): ppm
   - 2.05: ( s, 6H, -CH₃-C=C- )
   - 4.53: ( s, 1H, -CH-Ph )
   - 7.13: ( t , J = 1.2 Hz. , 1H , H₄ of imidazole ring )
   - 7.2-7.7: ( m, 4H , phenyl ring )
   - 7.76: ( t , J = 1.2 Hz. , 1H , H₅ of imidazole ring )
   - 8.27: ( t , J = 1.2 Hz. , 1H , H₂ of imidazole ring )
   - 9.6: ( bs , 1H, -NH )

By the same procedure the following compounds can be prepared:
4-(4-1H-imidazol-1-yl-phenyl)-2,6-dimethyl-1,4-dihydropyridine-3,5-dicarbonitrile:
m.p. = 230°C dec.
Microanalysis:
Found: C 69.75; H 5.10; N 22.09
Calculated for C₁₈H₁₅N₅: C 71.70; H 5.02; N 23.24
¹H-NMR ( 200MHz. , DMSO ): ppm
   - 2.03: ( s, 6H, -CH₃-C=C- )
   - 4.48: ( s, 1H , -CH-Ph )
   - 7.09: ( t , J = 1.2 Hz. , 1H , H₄ of imidazole ring )
   - 7.39-7.65: ( m, 4H, phenyl ring )
   - 7.72: ( t , J = 1.2 Hz. , 1H , H₅ of imidazole ring )
   - 8.23: ( t , J = 1.2 Hz. , 1H , H₂ of imidazole ring )
   - 9.55: ( bs , 1H , -NH )
4-[3-(1,2,4)triazol-1-yl-phenyl]-2,6-dimethyl-1,4-dihydropyridine-3,5-dicarbonitrile :
m.p.=270-274°C
Microanalysis:
Found: C 67.12; H 4.85; n 27.25
Calculated for C₁₇H₁₄N₆: C 67.54; H 4.67; N 27.79
¹H-NMR ( 200MHz. , DMSO ): ppm
   - 2.04: ( s , 6H, -CH₃-C=C- )
   - 4.57: ( s , 1H , -CH-Ph )
   - 7.3-7.9: ( m, 4H, phenyl ring )
   - 8.24-9.31: ( two s, 2H, tiazole ring )
   - 9.6: ( bs , 1H , -NH )

### EXAMPLE II

A mixture of 3-(1H-imidazol-1-yl)benzaldehyde (0.500g., 0.003 moles), ethyl acetoacetate (0.417g., 0.0032 moles) and 3-aminocrotonitrile (0.263g., 0.0032 moles) in absolute ethanol (10ml) is refluxed for 9 hours. The reaction mixture is evaporated under vacuum, diluted with water, extracted with CH₂Cl₂ and dried over Na₂SO₄. The residue is purified by silica gel column ( eluant: CH₂Cl₂/ EtOH = 95 / 5 ) giving a waxy solid wich after crystallization from ethyl acetate afforded 0.350g. (34%) of ±4-(3-1H-imidazol-1-yl-phenyl)-2,6-dimethyl-1,4-dihydropyridine-3-carbonitrile-5-carboxylic acid ethyl ester, m.p.=195-205°C.
Microanalysis:
Found: C 68.90; H 5.84; N 16.04
Calculated for C₂₀H₂₀N₄O₂: C 68.95; H 5.79; N 16.08
¹H-NMR ( 200MHz. , DMSO ): ppm
   - 1.06: ( t , 3H, -COOCH₂CH₃ )
   - 2.02-2.26: ( two s, 6H, -CH₃-C=C- )
   - 3.8-4.0: ( m , 2H , -COOCH₂- )
   - 4.56: ( s , 1H , -CH-Ph )
   - 7.09: ( s, 1H , H₄ of imidazole ring )
   - 7.1-7.5: ( m , 4H , phenyl ring )
   - 7.67: ( s , 1H , H₅ of imidazole ring )
   - 8.19: ( s, 1H, H₂ of imidazole ring )
   - 9.20: ( s , 1H , -NH )

By the same procedure the following compounds can be prepared :
±4-(3-1H-imidazol-1-yl-phenyl)-2,6-dimethyl-1,4-dihydropyridine-3-carbonitrile-5-carboxylic acid isopropyl ester; and
±4-[3-(1,2,4)triazol-1-yl-phenyl]-2,6-dimethyl-1,4-dihydropyridine-3-carbonitrile-5-carboxylic acid ethyl ester.

### EXAMPLE III

4-(3-1H-imidazol-1-yl-phenyl)-2,6-dimethyl-1,4-dihydropyridine-3,5-dicarbonitrile (0.301g., 0.001 moles) is dissolved in glacial acetic acid (5ml) and the solution added of NaNO₃ (0.340g., 0.004 moles) is refluxed for 30 min under stirring. After cooling, the reaction mixture is diluted with water, extracted three times with CH₂Cl₂ , dried over Na₂SO₄ and evaporated to dryness under vacuum. The residue is purified by silica gel column ( eluant : CH₂Cl₂ / EtOH = 98 / 2 ) giving a waxy solid wich is mixed with Et₂O The resulting precipitate is collected by filtration yielding 0.194g. (65%) of 4-(3-1H-imidazol-1-yl-phenyl)-2,6-dimethylpyridine-3,5-dicarbonitrile, m.p. = 215-218°C.
Microanalysis:
Found: C 71.56; H 4.41; N 22.97
Calculated for C₁₈H₁₃N₅: C 72.22; H 4.38; N 23.39
¹H-NMR (200MHz, DMSO): ppm
   - 2.81: ( s , 6H , -CH₃-C=C- )
   - 7.17: ( s , 1H , H₄ of imidazole ring )
   - 7.5-8.1: ( m , 5H , phenyl ring + H₅ of imidazole ring)
   - 8.35: ( s, 1H, H₂ of imidazole ring)

By the same procedure the following compounds can be prepared :
4-(3-1H-imidazol-1-yl-phenyl)-2,6-dimethylpyridine-3-carbonitrile-5-carboxylic acid ethyl ester;
4-[3-(1,2,4)triazol-1-yl-phenyl]-2,6-dimethylpyridine-3-carbonitrile-5-carboxylic acid ethyl ester; and
4-[3-(1,2,4)triazol-1-yl-phenyl]-2,6-dimethylpyridine-3,5-dicarbonitrile :
m.p. = 197.5-201°C
Microanalysis:
Found: C 67.14; H 4.07; N 27.43
Calculated for C₁₇H₁₂N₆: C 67.99; H 4.02; N 27.98
¹H-NMR (200MHz, DMSO): ppm
   - 2.79: ( s , 6H , -CH₃-C=C- )
   - 7.6-8.2: ( m , 4H , phenyl ring )
   - 8.39-9.37: ( two s , 2H , triazole ring );

### EXAMPLE IV

NaH (55% mineral oil dispersion) is added to a stirred solution of 4-(3-1H-imidazol-1-yl-phenyl)-2,6-dimethyl-1,4-dihydropyridine-3,5-dicarbonitrile (0.301g., 0.001 moles) in DMF (2ml) at room temperature under nitrogen atmosphere. Then methyl iodide (0.270g., 0.0019 moles) is added to the mixture. After 3 hours the reaction is treated with water, extracted with CH₂Cl₂ and dried over Na₂SO₄. The residue is purified by silica gel column ( eluant : CH₂Cl₂/ EtOH = 95 / 5 ) giving a waxy solid wich is mixed with Et₂O. The resulting precipitate is collected by filtration yielding 0.182g. (58%) of 4-(3-1H-imidazol-1-yl-phenyl)-1,2,6-trimethyl-1,4-dihydropyridine-3,5-dicarbonitrile,
m.p.=238-245°C dec.
Microanalysis:
Found: C 71.81; H 5.51; N 21.97
Calculated for C₁₉H₁₇N₅: C 72.35; H 5.43; N 22.2
¹H-NMR ( 200MHz. , DMSO ): ppm
   - 2.30: ( s , 6H , -CH₃-C=C- )
   - 3.22: ( s , 3H , -N-CH₃ )
   - 4.41: ( s, 1H, -CH-Ph )
   - 7.1-7.5: ( m, 6H, phenyl ring + H₄ , H₅ of imidazole ring )
   - 7.85: ( t, 1H , H₂ of imidazole ring )

By the same procedure the following compound can be prepared:
4-[3-(1,2,4)triazol-1-yl-phenyl]-1,2,6-trimethyl-1,4-dihydropyridine-3,5-dicarbonitrile.

### EXAMPLE V

4-(3-1H-imidazol-1-yl-phenyl)-2,6-dimethyl-1,4-dihydropyridine-3,5-dicarbonitrile (0.1g.) is dissolved in isopropyl alcohol (2ml) and treated with isopropanolic HCl. The solution is evaporated to dryness and the residue crystallized from ethylacetate/ethanol (3:1) mixture. The salt 4-(3-1H-imidazol-1-yl-phenyl)-2,6-dimethyl-1.4-dihydropyridine-3,5-dicarbonitrile hydrochloride is obtained in quantitative yield.

### EXAMPLE VI

Tablets, each weighing 150mg. and containing 50mg. of the active substance are manufactered as follows:

| Composition (for 10.000 tablets) | |
|---|---|
| 4-(3-1H-imidazol-1-yl-phenyl)-2,6-dimethyl-1,4-dihydropyridine-3,5-dicarbonitrile | 500 g. |
| Lactose | 710 g. |
| Corn starch | 237.5 g. |
| Talc powder | 37.5 g. |
| Magnesium stearate | 15 g. |

4-(3-1H-imidazol-1-yl-phenyl)-2,6-dimethyl-1,4-dihydropyridine-3,5-dicarbonitrile lactose and a half of the corn starch are mixed; the mixture is then forced through a sieve of 0.5 mm openings. Corn starch (18g.) is suspended in warm water (180ml). The resulting paste is used to granulate the powder. The granules are dried, comminuted on a sieve size 1.4 mm, then the remaining quantity of starch, talc and magnesium is added, carefully mixed, and processed into tablets using punches of 8 mm diameter.

### EXAMPLE VII

Capsules, each dosed at 0.5g. and containing 50mg. of the active substance can be prepared.

| Composition for 200 capsules: | |
|---|---|
| 4-(3-1H-imidazol-1-yl-phenyl)-2,6-dimethyl-1,4-dihydropyridine-3,5-dicarbonitrile | 10 g. |
| Lactose | 80 g. |
| Corn starch | 5 g. |
| Magnesium stearate | 5 g. |

This formulation is encapsulated in two-piece hard gelatin capsules and dosed at 0.5g. for each capsule.

### EXAMPLE VIII

By the usual pharmaceutical technique, suppositories having the following composition can be prepared:

| | |
|---|---|
| 4-(3-1H-imidazol-1-yl-phenyl)-2,6-dimethyl-1,4-dihydropyridine-3,5-dicarbonitrile | 0.05 g. |
| Lecithin | 0.07 g. |
| Cacao butter | 0.88 g. |

### EXAMPLE IX

### Intravenous injection 50 mg/ml.

An injectable pharmaceutical preparation can be manufactured by dissolving 50g. of 4-(3-1H-imidazol-1-yl-phenyl)-2,6-dimethyl-1.4-dihydropyridine-3,5-dicarbonitrile hydro chloride in water for injection (1000ml) and sealing in ampoules of 1-10ml.

## Claims

1. A compound of formula (I) or (II) 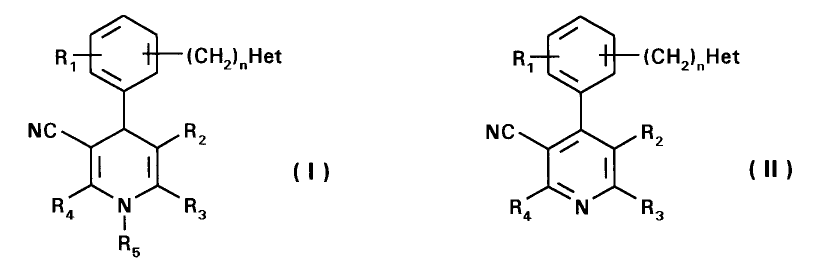 wherein:
Het is 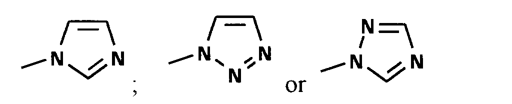 n is zero or 1;
R₁ is hydrogen, halogen, trihalomethyl or C₁-C₃ alkyl;
R₂ is cyano or COOR, wherein R is C₁-C₆ alkyl either unsubstituted or omega substituted by C₁-C₃ alkoxy;
each of R₃ and R₄, which may be the same or different, is a C₁-C₄ alkyl group, and
R₅ is hydrogen or a C₁-C₄ alkyl group, or a pharmaceutically acceptable salt thereof.

2. A compound according to claim 1, wherein
n is zero, R₁ is hydrogen, Het is 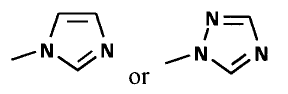 and R₂, R₃, R₄, and R₅ are as defined in claim 1, or a pharmaceutically acceptable salt thereof.

3. A compound selected from:
4-(3-1H-imidazol-1-yl-phenyl)-2,6-dimethyl-1,4-dihydropyridine-3,5-dicarbonitrile;
4-(4-1H-imidazol-1-yl-phenyl)-2,6-dimethyl-1,4-dihydropyridine-3,5-dicarbonitrile;
4-(3-1H-imidazol-1-yl-phenyl)-2,6-dimethyl-1,4-dihydropyridine-3-carbonitrile-5-carboxylic acid ethyl ester;
4-(3-1H--imidazol-1-yl-phenyl)-2,6-dimethyl-1,4-dihydropyridine-3-carbonitrile-5-carboxylic acid isopropyl ester;
4-[3-(1,2,4)triazol-1-yl-phenyl]-2,6-dimethyl-1,4-dihydropyridine-3,5-dicarbonitrile;
4-[3-(1,2,4)triazol-1-yl-phenyl]-2,6-dimethyl-1,4-dihydropyridine-3-carbonitrile-5-carboxylic acid ethyl ester;
4-(3-1H-imidazol-1-yl-phenyl)-2,6-dimethylpyridine-3,5-dicarbonitrile;
4-(3-1H-imidazol-1-yl-phenyl)-2,6-dimethylpyridine-3-carbonitrile-5-carboxylic acid ethyl ester;
4-[3-(1,2,4)triazol-1-ylphenyl]-2,6-dimethylpyridine-3,5-dicarbonitrile;
4-[3-(1,2,4)triazol-1-yl-phenyl]-2,6-dimethylpyridine-3-carbonitrile-5-carboxylic acid ethyl ester;
4-(3-1H-imidazol-1-yl-phenyl)-1,2,6-trimethyl-1,4-dihydropyridine-3,5-dicarbonitrile; and
4-[3-(1,2,4)triazol-1-yl-phenyl]-1,2,6-trimethyl-1,4-dihydropyridine-3,5-dicarbonitrile;
either as single isomer or as a mixture thereof or a pharmaceutically acceptable salt thereof.

4. A process for the preparation of a compound of formula (I) or (II), as defined in claim 1, or a salt thereof the process comprising:
a) reacting a compound of formula (III) 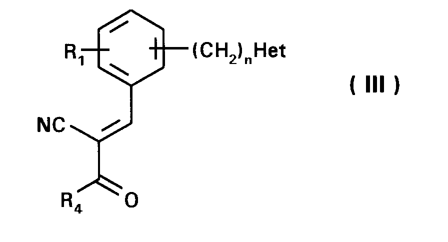 wherein
R₁, Het, n, and R₄ are as defined in claim 1, with a compound of formula (IV) 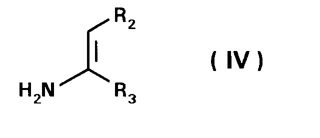 wherein
R₂ and R₃ are as defined in claim 1, thus obtaining a compound of formula (I) in which R₅ is hydrogen; or
b) reacting a compound of formula (III), as defined above, with a compound of formula (V) 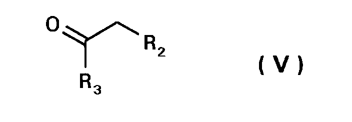 wherein
R₂ and R₃ are as defined in claim 1, in the presence of an ammonium salt or hydroxide, thus obtaining a compound of formula (I) in which R₅ is hydrogen; or
c) reacting a compound of formula (VI) 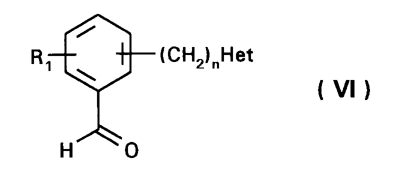 wherein R₁, n and Het are as defined in claim 1, with a compound of formula (IV), as defined above, and a compound of formula (VII), together, 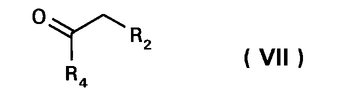 wherein, R₂ and R₄ are as defined in claim 1 and in at least one of the compounds (IV) and (VII) R₂ is cyano, thus obtaining a compound of formula (I) wherein R₅ is hydrogen; or
d) reacting a compound of formula (VI), as defined above, with a compound of formula (V), as defined above, and a compound of formula (VII), as defined above, together, wherein in at least one of the compounds (V) and (VII) R₂ is cyano, in the presence of ammonium salt or hydroxide, thus obtaining a compound of formula (I) wherein R₅ is hydrogen; or
e) reacting a compound of formula (VI), as defined above, with at least two equivalents of a compound of formula (IV) , as defined above, wherein R₂ is a cyano group, thus obtaining a compound of formula (I), wherein R₂ is cyano, R₃ and R₄, being as defined in claim 1, are the same and R₅ is hydrogen; or
f) alkylating a compound of formula (I), as defined in claim 1, wherein R₅ is hydrogen, thus obtaining a compound of formula (I) wherein R₅ is C₁-C₄ alkyl; or
g) oxidizing a compound of formula (I), as defined in claim 1, wherein R₅ is hydrogen, thus obtaining a compound of formula (II); and, if desidered, converting a compound of the invention into another compound of the invention; and/or, if desired, converting a compound of formula (I) or (II) into a pharmaceutically acceptable salt thereof and/or, if desired, converting a salt into a free compound, and/or, if desired, separating a mixture of isomers of formula (I) or (II) into the single isomers.

5. A pharmaceutical composition comprising a pharmaceutically acceptable carrier and/or diluent and, as an active compound, a compound of formula (I) or (II), according to claim 1, or a pharmaceutically acceptable salt thereof.

6. A compound of formula (I) or (II), as defined in claim 1, or a pharmaceutically acceptable salt thereof for use as an active therapeutic substance.

7. A compound of formula (I) or (II), as defined in claim 1, or a pharmaceutically acceptable salt thereof for use as a steroidal aromatase inhibitor.

8. A compound of formula (I) or (II), as defined in claim 1, or a pharmaceutically acceptable salt thereof for use in the treatment or prevention of an estrogen-dependent disease.

9. A compound of formula (I) or (II), as defined in claim 1, or a pharmaceutically acceptable salt thereof, for use in the treatment or prevention of breast, endometrial, ovarian or pancreatic cancers, gynecomastia, benign breast disease, endometriosis, polycystic ovarian disease or precocious puberty.

10. A compound of formula (I) or (II), as defined in claim 1, or a pharmaceutically acceptable salt thereof for use in the treatment or prevention of prostatic hyperplasia, in the treatment of male infertility or for female fertility control.
